# EUROPEAN PATENT APPLICATION

(11) **EP 4 008 318 A1**
(43) Date of publication of application: **08.06.2022**
(21) Application number: 21211924.2
(22) Date of filing: 02.12.2021
(51) Int. Cl.: A61K 9/20, A61K 9/28

(54) **A PROCESS FOR TABLET FORMULATIONS COMPRISING AMORPHOUS DAPAGLIFLOZIN AND METFORMIN HYDROCHLORIDE**

(30) Priority: 03.12.2020 TR 202019592; 08.06.2021 TR 202109380
(71) Applicant: SANOVEL ILAC SANAYI VE TICARET A.S., Istanbul 34460 (TR)
(72) Inventor: PEHLIVAN AKALIN, Nur, 34460 Istanbul (TR); SÜNEL, Fatih, 34460 Istanbul (TR); OZDEN, Aydan, 34460 Istanbul (TR)
(74) Representative: Sevinç, Erkan

(57) **Abstract**

The present invention relates to a process for the preparation of a film coated tablet formulation comprising amorphous dapagliflozin and metformin hydrochloride. The process is wet-granulation which is simple, rapid, cost effective, time-saving and industrially convenient process and is created to eliminate the disadvantages of both active ingredients.

## Description

### Field of the Invention

The present invention relates to a process for the preparation of a film coated tablet formulation comprising amorphous dapagliflozin and metformin hydrochloride. The process is wet-granulation which is simple, rapid, cost effective, time-saving and industrially convenient process and is created to eliminate the disadvantages of both active ingredients.

### Background of the Invention

Diabetes mellitus, known as diabetes, is a lifelong chronic disease resulting from the pancreas's inability to produce enough insulin or the body's ability to use insulin effectively, and it continues with the reduction of insulin-producing cells. Blood glucose rises with absent or non-functioning insulin hormone. The resulting high blood sugar causes classical symptoms such as polyuria (frequent urination), polydipsia (increased thirst) and polyphagia (increased hunger). Currently, there are three types of diabetes: type 1 diabetes (type 1), type 2 diabetes (type 2) and gestational diabetes. Gestational diabetes is seen in pregnant women who develop high blood sugar levels. Type 1 diabetes is caused by the body's inability to produce insulin, requiring insulin injection. Finally, with type 2 diabetes, the body either resists the effects of insulin or does not produce enough insulin to maintain a normal glucose level. Of these three types of diabetes, type 2 diabetes is the most common type, affecting more than 171 million people worldwide.

Dapagliflozin is a sodium-glucose cotransporter 2 inhibitor (SGLT2). SGLT2 is a carrier responsible for the reabsorption of most of the glucose from the lumen of the renal tubule. SGLT2 is expressed in proximal renal tubules. By inhibiting SGLT2, dapagliflozin reduces the reabsorption of the filtered glucose and lowers the renal threshold for glucose. This improves urinary glucose excretion and blood glucose control. Dapagliflozin, also known as (1S)-1,5-Anhydro-1-C-[4-chloro-3-[(4-ethoxyphenyl)methyl]phenyl]-D-glucitol or (2S,3R,4R,5S,6R)-2-(3-(4-etoxybenzyl)-4-chlorophenyl)-6-hydroxymethyltetrahydro-2H-pyran-3,4,5-triol is represented by the structure of Formula I.

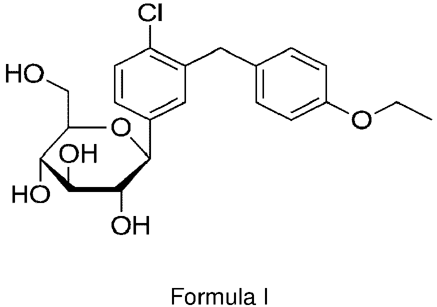

Dapagliflozin was first disclosed in patent US 6515117 (2003, Bristol-Myers Squibb).

Metformin is an antihyperglycemic agent that improves glucose tolerance and lowers both basal and postprandial plasma glucose levels by different mechanisms than other oral antidiabetic agents. Metformin decreases hepatic gluconeogenesis, decreases intestinal absorption of glucose, and improves insulin sensitivity by increasing peripheral glucose uptake and utilization. With metformin therapy, insulin secretion remains unchanged while fasting insulin levels and day-long plasma insulin response may actually decrease. Metformin, also known as N, N-dimethylimidodicarbonimidic diamide or 1,1-Dimethylbiguanide or N, N-dimethyldiguanide or N, N-Dimethylguanylguanidine, is represented by the chemical structure in Formula II.

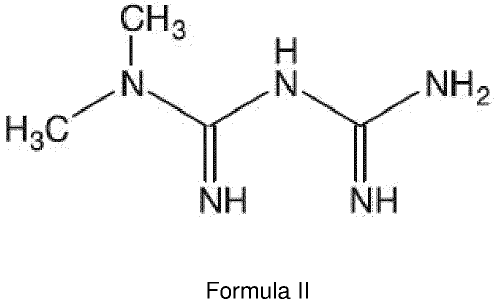

Metformin was first disclosed in patent US 3174901 (1965, Jan Marcel Didier Aron-Samuel).

EP2498758B1 discloses bilayer tablets comprising a first layer, wherein the first layer is extended release metformin and a second layer, wherein the second layer is immediate release dapagliflozin molecule.

EP2498759B1 discloses a process for the preparation of an immediate release formulation comprising a combination of dapagliflozin and metformin.

WO 2017/098481 discloses an effervescent combination comprising metformin with retained carbon dioxide content of at least 90% of the input blend and a different antidiabetic agent.

The dapagliflozin is hygroscopic in nature. It absorbs moisture and forms sticky lumps which are difficult to process and handle, and which may ultimately lead to content uniformity issues in the dosage form. The low solubility and stability of dapagliflozin base as compared to its solvates may lead to poor bioavailability of the drug.

Although several solid forms comprising dapagliflozin are known in the art, finding a good or even the optimal form with regard to bioavailability and safety remains a considerable challenge, in particular when the compound forms many polymorphic forms. Not all solid forms comprising dapagliflozin are equally suitable with regard to stability, flow properties, compressibility, dissolution rate.

The invention provides an amorphous form of dapagliflozin to improve dissolution profile. Although, amorphous form is better soluble than crystalline form, amorphous form of dapagliflozin has disadvantages such as poor physical stability resulting in crystallization and poor chemical stability resulting in degradation.

Metformin is a very poorly compressible active substance and metformin presents in high amounts in a composition. The problem causes some content uniformity problems.

There is thus still a need for a process for the preparation of a film coated tablet comprises amorphous dapagliflozin and metformin that provides a tablet having the desired stability, dissolution profile, hardness and compressibility, in another words the disadvantages seen in the active substances will able to overcome.

### Detailed Description of the Invention

The main object of the present invention is to eliminate problems caused by active substances and bringing additional advantages to the relevant prior art.

Another object of the present invention is to provide a powder mixture which prepared with the use of preferred excipients and the production process is homogeneous, compressible and flowable with ideal particle properties. It has good friability and no capping problem is observed. It has a suitable dissolution profile and is stable throughout the shelf life.

Another object of the present invention is to provide a process for a film coated tablet formulation comprising amorphous dapagliflozin and metformin hydrochloride having the desired stability, dissolution profile, hardness and compressibility.

In this invention, combining more than one molecule in one dosage form increases the patient's compliance. However, while this combination is increasing the patients' quality of life, combining more than one molecule in one dosage form also reduces side effects.

In view of the above art, there is still a need for an effective solid pharmaceutical formulation comprising of a form of dapagliflozin. The invention provides a process for a film coated tablet comprising an amorphous form of dapagliflozin and metformin HCI. Although amorphous forms are sometimes better soluble than crystalline forms, they are often not the preferred form because of water activity and/or stability reasons. However, the present invention eliminates all aforesaid problems and bring additional advantages to the relevant prior art.

Using amorphous form of dapagliflozin is to improve dissolution. Amorphous solids are commonly used for improving dissolution and oral absorption of poorly soluble compounds because they exhibit higher apparent solubility values and faster dissolution rates compared with crystalline materials. However, they also have disadvantages, such as poor physical stability, which can result in crystallization, and poor chemical stability, which can lead to degradation. Since they are metastable forms, development of amorphous compounds involves a greater understanding of the handling, storage, and processing conditions needed to prevent crystallization and maintain the amorphous state. Amorphous solid dispersions are defined as an amorphous API mixed with a polymer or mixtures of polymers. The polymer stabilizes the amorphous drug by lowering the chemical potential of the drug and increasing the activation energy needed for crystallization, which results in better physical and chemical stability, higher apparent solubility, and faster dissolution. For this aim, especially using of Polyvinylpyrrolidone having an average K value range 11-95 polymers provides a tablet having the desired stability, dissolution profile, hardness and compressibility, in another words the disadvantages seen in the active substances will able to overcome.

Amorphous dapagliflozin is used small proportion that can lead to considerable problems during the manufacture of the composition with regard to the uniformity of the content of active agent in the individual composition units. Because of problems uniformity of the content, the active substance may interact with several excipients. It reflects that content uniformity play important role in the dissolution of the drug. Especially for this reason, we used the binder both in the dry mix and in the preparation of granulation solution. We saw that the use of a binder both in the dry mix and in the preparation of granulation solution surprisingly achieved the desired stability, content uniformity, flowability and homogenity. According to an embodiment of the present invention, the film coated tablet is prepared wet granulation. So, an easy method was created to eliminate the disadvantages of both active ingredients. Wet granulation process efficiently counteracts the segregation of active agents, so it is observed that the desired stability, tablet hardness, content uniformity and compressibility.

According to an embodiment of the present invention, a process for the preparation of a film coated tablet comprises amorphous dapagliflozin and metformin hydrochloride comprising the following steps:
a) Mixing amorphous dapagliflozin, metformin hydrochloride, at least one binder, at least one disintegrant and at least one filler,
b) Dissolving at least binder in a solvent to obtained a solution,
c) Granulating the mixture at step (a) with the solution at step (b).

So, an easy method is created to eliminate the disadvantages of both active ingredients and also the method is simple and cost-effective method. Also, this process helps to provide the desired dissolution profile.

According to an embodiment of the present invention, the amount of amorphous dapagliflozin is 0.05% to 3.0% by weight, preferably 0.1% to 2.0% by weight in the total composition.

According to an embodiment of the present invention, the amount of metformin HCI is 65.0% to 80.0% by weight, preferably 68.0% to 75.0% by weight in the total composition.

In the process, metformin HCI and dapagliflozin are put into the tank at the same time with at least one disintegrant and at least one filler and at least one binder. Thus, the desired mixture can be achieved without loss of active ingredient.

According to one embodiment of the invention, at least one binder is dissolved in a solvent and a binder solution is obtained. For this reason, it ensures that active agents and excipients adhere to each other in an ideal way and provides homogeneous granule units. In this way, possible blend uniformity and content uniformity problems that may be seen in the product are prevented.

Suitable binders are selected from the group comprising polyvinylpyrrolidone, hydroxypropyl methylcellulose, lactose anhydrous, pregelatinized starch, calcium carbonate, calcium phosphate dibasic, calcium phosphate tribasic, calcium sulphate, hydroxypropylcellulose, carboxymethylcellulose, methyl cellulose, gelatin I, ethyl cellulose or mixtures thereof. According to an embodiment of the present invention, the binder is polyvinylpyrrolidone having an average K value range 10-100. Especially, polyvinylpyrrolidone K90 F or polyvinylpyrrolidone VA 64 is used as binder.

Examples for polyvinylpyrrolidone;

| **Polyvinylprolidone Types** | **K Value** | **Typical Viscosity Range** |
|---|---|---|
| Polyvinylprolidone 12 | 10-14 | 1.3-2.3 mPa.s |
| Polyvinylprolidone 17 | 15-18 | 1.5-3.5 mPa.s |
| Polyvinylprolidone 25 | 20-27 | 3.5-5.5 mPa.s |
| Polyvinylprolidone 30 | 27-32 | 5.5-8.5 mPa.s |
| Polyvinylprolidone VA 64 | 25-70 | 100-500 mPa.s |
| Polyvinylprolidone K 90 F | 80-100 | 300-700 mPa.s |

According to an embodiment of the present invention, the binder is polyvinylpyrrolidone K90 F or polyvinylpyrrolidone VA 64 or mixtures thereof. Especially, the use of polyvinylpyrrolidone polymers as binders has been observed to inhibit the crystallization of dapagliflozin in amorphous form. It is seen that the combination of polyvinylprolidone VA 64 and polyvinylprolidone K90 F gives very successful results in the stabilization of our formulation.

According to an embodiment of the present invention, the amount of binders is 2.0% to 10.0% by weight, preferably 4.0% to 8.0% by weight in the total composition.

Suitable fillers are selected from the group comprising microcrystalline cellulose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

According to an embodiment of the present invention, the filler is microcrystalline cellulose.

According to an embodiment of the present invention, the amount of fillers is 10.0% to 25.0% by weight, preferably 14.0% to 22.0% by weight in the total composition.

Suitable disintegrants are selected from the group comprising croscarmellose sodium, sodium starch glycollate, crospovidone, sodium alginate, gums, starch and magnesium aluminum silicate or a mixture thereof.

According to an embodiment of the present invention, the disintegrant is croscarmellose sodium. With this disintegrant being preferred, the formulation has a suitable in vitro and in vivo profile.

According to an embodiment of the present invention, the amount of disintegrants is 1.0% to 5.0% by weight in the total composition.

According to an embodiment of the present invention, solvents are selected from the group comprising pure water, propylene glycol, glycerin, ethanol, polyethylene glycol or mixtures thereof.

According to an embodiment of the present invention, the solvent is pure water.

According to an embodiment of the present invention, wet granulation is used at step (c).

The advantages of the present invention are even more significant, as the problem of homogeneity is even more likely to occur when two active substances are incorporated in one final dosage form, especially when two actives is used very different regarding the amount. Improved content uniformity efficiently contributes to a marked increase in bioavailability. Improved content uniformity also favors to avoid toxicity in the otherwise possible event that the amount of drug substance would be too high.

According to an embodiment of the present invention, a process for the preparation of a film coated tablet comprises dapagliflozin and metformin hydrochloride comprising the following steps:
a) Mixing dapagliflozin, metformin hydrochloride, polyvinylpyrrolidone (K90 F), croscarmellose sodium and microcrystalline cellulose,
b) Dissolving polyvinylpyrrolidone (VA 64) in pure water to obtained a solution,
c) Granulating the mixture at step (a) with the solution at step (b)

According to one embodiment of the present invention, the process for the preparation of the film coated tablet further comprises the following steps:
d) Drying the mixture in fluid bed dryer, then sieving the mixture,
e) Adding at least one lubricant and mixing,
f) Compressing the mixture to form a tablet,
g) Coating tablets with film coating agents.

It has been observed that the compressibility properties of the granules dried in a fluid bed dryer are quite good. Ideal printing properties allowed to work with high printing speed. Tablet printing times are minimized as it allows to work with high printing speed.

Suitable lubricants are selected from the group comprising magnesium stearate, silica colloidal anhydrous, sodium stearyl fumarate, talc, polyethylene glycol, stearic acid, aluminum silicate or mixtures thereof.

According to an embodiment of the present invention, the lubricant is magnesium stearate.

According to an embodiment of the present invention, the amount of lubricants is 0.1% to 5.0% by weight, preferably 0.1 to 3.0% by weight in the total composition.

Film coating is important for the present invention, because dapagliflozin absorbs moisture, film coating protect the composition against the moisture and light to maintain the stability. the film coated tablet comprises coating agents for film coating.

Suitable coating agents are selected from the group comprising polymethacrylates, hydroxypropyl methylcellulose, lactose monohydrate, talc, hydroxypropyl cellulose, polyvinyl alcohol (PVA), polyethylene glycol (PEG), talc, glycerine, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), Iron oxide all kinds of Opadry^{®}, pigments, dyes, titanium dioxide, coloring agent or mixtures thereof.

According to an embodiment of the present invention, the coating agents are polyvinyl alcohol, talc, titanium dioxide, polyethylene glycol, iron oxide.

According to an embodiment of the present invention, the amount of coating agents is 1.0% to 5.0% by weight by weight in the total composition.

According to an embodiment of the present invention, the film coated tablet of the present invention comprises dapagliflozin and metformin HCI, polyvinylpyrrolidone K90 F, polyvinylpyrrolidone VA 64, microcrystalline cellulose, croscarmellose sodium, magnesium stearate.

### Example 1: The film coated tablet

| **Ingredients** | **Ingredients in one tablet (%) (by weight)** |
|---|---|
| Amorphous dapagliflozin | 0.05-3.0 |
| Metformin HCI | 65.0-80.0 |
| Polyvinylpyrrolidone VA 64 | 1.0 - 5.0 |
| Polyvinylpyrrolidone K90 F | 1.0 - 5.0 |
| Microcrystalline cellulose | 10.0 - 25.0 |
| Croscarmellose sodium | 1.0 - 5.0 |
| Magnesium stearate | 0.1 -5.0 |
| Film coating agents (polyvinyl alcohol partially hydrolyzed, talc, titanium dioxide, polyethylene glycol, iron oxide yellow or red) | 1.0 - 5.0 |
| **Total** | **100** |

### Example 2: The film coated tablet

| **Ingredients** | **Ingredients in one tablet (%) (by weight)** |
|---|---|
| Amorphous dapagliflozin | 0.4 |
| Metformin HCI | 70.3 |
| Polyvinylpyrrolidone VA 64 | 3.5 |
| Polyvinylpyrrolidone K90 F | 1.4 |
| Microcrystalline cellulose | 18.4 |
| Croscarmellose sodium | 2.1 |
| Magnesium stearate | 1.0 |
| Film coating agents (polyvinyl alcohol partially hydrolyzed, talc, titanium dioxide, polyethylene glycol, iron oxide yellow or red) | 3.0 |
| **Total** | **100** |

A process for example 1 or 2;
a) Mixing amorphous dapagliflozin, metformin hydrochloride, polyvinylpyrrolidone K90 F, croscarmellose sodium and microcrystalline cellulose,
b) Dissolving polyvinylpyrrolidone VA 64 in pure water to obtained a solution,
c) Granulating the mixture at step (a) with the solution at step (b)
d) Drying the mixture in fluid bed dryer, then sieving the mixture,
e) Adding magnesium stearate and mixing,
f) Compressing the mixture to form a tablet,
g) Coating tablets with film coating agents.

## Claims

1. A process for the preparation of a film coated tablet comprises amorphous dapagliflozin and metformin hydrochloride comprising the following steps:
a) Mixing amorphous dapagliflozin, metformin hydrochloride, at least one binder, at least one disintegrant and at least one filler,
b) Dissolving at least binder in a solvent to obtained a solution,
c) Granulating the mixture at step (a) with the solution at step (b).

2. The process for the preparation of a film coated tablet according to claim 1, wherein binders are selected from the group comprising polyvinylpyrrolidone, hydroxypropyl methylcellulose, lactose anhydrous, pregelatinized starch, calcium carbonate, calcium phosphate dibasic, calcium phosphate tribasic, calcium sulphate, hydroxypropylcellulose, carboxymethylcellulose, methyl cellulose, gelatin I, ethyl cellulose or mixtures thereof.

3. The process for the preparation of a film coated tablet according to claim 2, wherein the binder is polyvinylpyrrolidone K90 F or polyvinylpyrrolidone VA 64 or mixtures thereof.

4. The process for the preparation of a film coated tablet according to claim 3, wherein the amount of binders is 2.0% to 10.0% by weight, preferably 4.0% to 8.0% by weight in the total composition.

5. The process for the preparation of a film coated tablet according to claim 1, wherein fillers are selected from the group comprising microcrystalline cellulose, lactose, starch, mannitol, calcium hydrogen phosphate dihydrate, dicalcium hydrogen phosphate anhydrate, calcium phosphate trihydrate, silicon dioxide, neutral pellets, magnesium carbonate, magnesium oxide, maltodextrin, maltose, medium chain triglycerides or mixtures thereof.

6. The process for the preparation of a film coated tablet according to claim 5, wherein the filler is microcrystalline cellulose.

7. The process for the preparation of a film coated tablet according to claim 1, wherein disintegrants are selected from the group comprising croscarmellose sodium, sodium starch glycollate, crospovidone, sodium alginate, gums, starch and magnesium aluminum silicate or a mixture thereof.

8. The process for the preparation of a film coated tablet according to claim 7, wherein the disintegrant is croscarmellose sodium.

9. The process for the preparation of a film coated tablet according to claim 1, wherein wet granulation is used at step (c).

10. The process for the preparation of a film coated tablet according to claim 1, wherein comprising the following steps:
a) Mixing dapagliflozin, metformin hydrochloride, polyvinylpyrrolidone K90 F, croscarmellose sodium and microcrystalline cellulose,
b) Dissolving polyvinylpyrrolidone VA 64 in pure water to obtained a solution,
c) Granulating the mixture at step (a) with the solution at step (b).

11. The process for the preparation of a film coated tablet according to claim 10, wherein further comprises the following steps:
d) Drying the mixture in fluid bed dryer, then sieving the mixture,
e) Adding at least one lubricant and mixing,
f) Compressing the mixture to form a tablet,
g) Coating tablets with film coating agents.
